# EUROPÄISCHE PATENTSCHRIFT

(11) **EP 1 575 886 B1**
(45) Veröffentlichungstag und Bekanntmachung des Hinweises auf die Patenterteilung: **20.02.2008**
(21) Anmeldenummer: 03778308.1
(22) Anmeldetag: 11.11.2003
(51) Int. Cl.: C07C 29/04, C07C 31/12

(54) **VERFAHREN ZUR HERSTELLUNG VON TERT.-BUTANOL MITTELS REAKTIVREKTIFIKATION**
METHOD FOR PRODUCING TERT-BUTANOL BY MEANS OF REACTIVE RECTIFICATION
PROCEDE DE PREPARATION DE TERT-BUTANOL PAR LA RECTIFICATION REACTIVE

(30) Priorität: 24.12.2002 DE 10260991
(43) Veröffentlichungstag der Anmeldung: 21.09.2005
(73) Patentinhaber: Evonik Oxeno GmbH, 45772 Marl (DE)
(72) Erfinder: REUSCH, Dieter, 45772 Marl (DE); BECKMANN, Andreas, 45657 Recklinghausen (DE); NIERLICH, Franz, 45768 Marl (DE); TUCHLENSKI, Axel, 69469 Weinheim (DE)
(74) Vertreter: Hirsch, Hans-Ludwig
(86) Internationale Anmeldenummer: PCT/EP2003/012535
(87) Internationale Veröffentlichungsnummer: WO 2004/058669

(56) Entgegenhaltungen:
- EP-A- 0 415 310
- EP-A- 0 466 954
- US-A- 5 258 560

## Beschreibung

Die Erfindung betrifft ein Verfahren zur Herstellung von tert.-Butanol (TBA) aus isobutenhaltigen Gemischen, mit Hilfe einer Reaktivrektifikation.

Tert.-Butanol (TBA) ist ein wichtiges großtechnisch hergestelltes Produkt und wird als Lösungsmittel und als Zwischenprodukt für die Herstellung von Methacrylsäuremethylester verwendet. Es ist Vorstufe für die Herstellung von Peroxiden, wie Peroxiketale, Perester oder Dialkylperoxide, mit mindestens einer tertiären Butylgruppe. Diese Verbindungen werden als Oxidationsmittel und als Starter für Radikalreaktionen, wie beispielsweise Olefinpolymerisation oder Vernetzung von Kunststoffen, eingesetzt. Als Zwischenstufe dient tert.-Butanol zur Gewinnung von reinem Isobuten aus Isobutengemischen. Darüber hinaus ist es ein Reagens zur Einführung von tertiären Butylgruppen. Seine Alkalisalze sind starke Basen, die in vielen Synthesen Verwendung finden.

TBA kann durch Oxidation von Isobutan hergestellt werden oder es fällt als Koppelprodukt bei der Epoxidierung von Olefinen mit tert.-Butylperoxid an. Solche Prozesse arbeiten gewöhnlich in der flüssigen Phase und können in zwei Gruppen unterteilt werden: a) Verfahren, bei denen die Umsetzung in einer wässrigen Katalysatorlösung erfolgt und b) heterogene katalytische Prozesse, bei denen feste, in der Reaktionsphase unlösliche Katalysatoren eingesetzt werden.

Homogene katalytische Verfahren verwenden als Katalysatoren Schwefelsäure, Heteropolysäuren, p-Toluolsulfonsäure oder andere starke Säuren. Diese Katalysatoren mit hoher Aktivität bilden mit dem Reaktionsprodukt gewöhnlich eine homogene Phase, sodass der Katalysator nicht mechanisch abgetrennt werden kann. Wird das tertiäre Butanol durch Destillation aus der Reaktionslösung gewonnen, so vermindert sich die Ausbeute durch Rückreaktion und die Bildung von Nebenprodukten.

Die Hydratisierung von Isobuten zu tert.-Butanol mit Hilfe fester, weder in den Edukten noch in den Produkten löslicher, saurer Katalysatoren hat den Vorteil, dass das Reaktionsgemisch säurefrei ist und ohne Verluste durch Rückspaltung oder durch andere Nebenreaktionen zu tert.-Butanol aufgearbeitet werden kann. Die Reaktion läuft an der Oberfläche des Katalysators ab. Damit eine Reaktion zustande kommt, müssen beide Reaktionspartner zur gleichen Zeit an der aktiven Stelle des Katalysators sein. Dies wird dadurch erschwert, dass Wasser und Isobuten bzw. ein Isobuten-haltiges Kohlenwasserstoffgemisch nicht miteinander mischbar sind. Um akzeptable Umsätze zu erhalten, werden häufig Solventien verwendet, die ein homogenes Gemisch aus Wasser und Isobuten-Einsatzgemisch ermöglichen.

In DE 30 31 702 wird Methanol für diesen Zweck als Lösungsmittel sowohl für Wasser als auch für Isobuten bzw. für ein Isobuten-haltiges Kohlenwasserstoffgemisch beschrieben. Als Produkte werden tert.-Butanol und Methyl-tert.-butylether nebeneinander erhalten. Hier ist nachteilig, dass das Lösungsmittel in einer zusätzlichen Trenneinheit vom gewünschten Produkt wieder abgetrennt werden muss und daher zusätzliche Apparate- und Betriebskosten nach sich zieht.

In EP 0 010 993 werden aliphatische Carbonsäuren mit 1 bis 6 C-Atomen als Lösungsmittel für beide Edukte eingesetzt. Dabei entstehen als Nebenprodukte die tertiären Butylester dieser Säuren. Diese müssen zu tert.-Butanol und Carbonsäuren hydrolysiert werden. Auch hier ist nachteilig, dass das Lösungsmittel in einer zusätzlichen Trenneinheit vom gewünschten Produkt wieder abgetrennt werden muss und daher zusätzliche Apparate- und Betriebskosten nach sich zieht.

In WO 99/33775 wird ein Verfahren zur Herstellung von tert.-Butanol durch Umsetzung einer Mischung, bestehend aus Wasser, tert.-Butanol und Isobuten bzw. einem isobutenhaltigen Kohlenwasserstoffgemisch, an einem Kationenaustauscherharz in einem Reihen-Multistufen-Reaktor beschrieben. Die Reaktionstemperatur in den einzelnen Reaktoren liegt unter 65 °C. Ein Teil des Zwischenprodukts aus dem ersten Reaktor wird in den Eingang des gleichen Reaktors zurückgeführt. Die Zirkulationsrate (die Menge an Zwischenproduktgemisch, die wieder in den ersten Reaktor zurückgeleitet wird, als Verhältnis zum Einsatzgemisch) beträgt hierbei 1,8 bis 10 und der Gewichtsanteil an tert.-Butylalkohol gegenüber dem Kohlenwasserstoffgemisch (Summe aus Isobuten und gegebenenfalls anderen Kohlenwasserstoffen) am Eingang des ersten Reaktors 0,5 bis 3,5. Das nicht zurückgeführte Gemisch aus dem ersten Reaktor durchströmt ohne Zwischeneinspeisung von Wasser zwei weitere Reaktoren im geraden Durchgang. Das Rohprodukt aus dem letzten Reaktor wird destillativ aufgearbeitet. Gegebenenfalls wird ein Teil des gewonnenen tert.-Butanol in den ersten Reaktor zurückgeführt. Nachteilig an diesem Verfahren ist die geringe Raum-Zeit-Ausbeute.

In DE 030 25 262 und US 6111148 werden Verfahren zur Herstellung von TBA aus Isobuten und Wasser offengelegt, bei dem das Zielprodukt, tert.-Butanol, als Lösungsvermittler zwischen Isobuten und Wasser eingesetzt wird. Dabei wird ein Gemisch aus Isobuten bzw. einem isobutenhaltigen Kohlenwasserstoffgemisch, Wasser und tert.-Butanol an einem stark sauren Ionenaustauscherharz in mehreren in Reihe geschalteten Reaktoren zu tert.-Butanol umgesetzt. Das den letzten Reaktor verlassende Reaktionsgemisch wird destilliert. Als Kopfprodukt fällt ein Gemisch aus nicht umgesetztem Isobuten und gegebenenfalls von unter Reaktionsbedingungen inerten Kohlenwasserstoffen aus dem Edukt an. Als Sumpfprodukt wird eine wässrige tert.-Butanollösung gewonnen. Ein Teil davon wird in den ersten Reaktor zurückgeführt.

Alle genannten Verfahren besitzen den Nachteil, dass wegen der thermodynamischen Gleichgewichtslage ein vollständiger Umsatz von Isobuten zu TBA nicht möglich ist. Demzufolge ist es auch nicht möglich, eine Isobuten enthaltende C₄-Kohlenwasserstofffraktion, z. B. Raffinat I, vollständig vom Isobuten zu befreien. Des weiteren haben herkömmliche Verfahren den Nachteil, dass die Reaktion bei niedrigen Temperaturen durchgeführt werden muss, um die Gleichgewichtslage in Richtung TBA zu verschieben. Dies hat allerdings langsame Reaktionsgeschwindigkeiten und somit große Reaktorvolumina zur Folge.

Zur Überwindung von Reaktionsgleichgewichten hat sich das Konzept der Reaktivdestillation in zahlreichen technisch bedeutsamen Reaktionen bewährt. Zu nennen sind hier Ethersynthesen (Methyl-tert.-butylether (MTBE), Ethyl-tert.-butylether (ETBE), Methyl-tert.-pentylether (TAME), Ethyl-tert.-pentylether (TAEE)), Veresterungen, Umesterungen, Isomerisierungen, Etherspaltungen oder Dehydratisierungen. Das Prinzip der Reaktivdestillation basiert auf der parallel zur Reaktion ablaufenden Destillation. Die Reaktionsprodukte werden selektiv aus dem Reaktionsraum abgezogen und somit aus dem Gleichgewicht entfernt.

In US-A 5,258,560 wird ein Verfahren zur Herstellung von Ethen beschrieben, bei dem ein Gemisch, das zu mindest ein C₄₊-Isoolefin enthält, und ein Alkohol durch eine katalytische Destillationszone geführt wird.

Die Herstellung tertiärer Alkohole, insbesondere Tertiäramylalkohol (tert.-Pentanol) und Tertiärbutylalkohol durch Reaktivdestillation in Gegenwart eines sauren Ionentauschers ist in EP-A-0 415 310 und DE 100 50 627 beschrieben. In EP-A-0 415 310 wird das Verfahren in einer Reaktivdestillationskolonne mit helikal gewickelten Gewebestrukturen durchgeführt. Dagegen verwendet man in DE 100 50 627 bevorzugt strukturierte Mehrzweckpackungen, wie sie beispielsweise im Handel erhältlich sind als Katapak® der Sulzer AG oder Montz Multipak der Firma Montz GmbH. Das Kolonnenkonzept ist bei beiden Verfahren gleich, der Abtriebsteil der Kolonne ist ausschließlich mit Destillationseinbauten befüllt während der Verstärkungsteil bevorzugt mit reaktiven Einbauten ausgerüstet ist. Sowohl in EP-A-Ö 415 310 als auch in DE 100 50 627 wird das C₄-Kohlenwasserstoffgemisch unterhalb der reaktiven Zone zugeführt, während oberhalb der reaktiven Zone das Wasser in die Kolonne gelangt. Nachteilig an dieser Betriebsweise ist, dass es insbesondere im oberen Kolonnenabschnitt ohne die lösungsvermittelnde Wirkung des tertiären Alkohols zu Entmischungen zwischen organischer und wässriger Phase kommt, was die Wirksamkeit der Destillation und insbesondere der Reaktion maßgeblich einschränkt, wodurch die Raum-Zeit-Ausbeute in der Reaktivdestillationskolonne vermindert wird. Zudem wird die Reaktionsgeschwindigkeit durch die Hydratisierung des Katalysators mit Wasser vermindert, da Isobuten als unpolare Verbindung nur noch langsam durch die Hydrathülle diffundieren kann.

DE 100 56 685 offenbart ein Verfahren zur Herstellung von Isobuten aus einem Iso- und n-Buten-haltigen C₄-Strom, bei dem zunächst in einer Reaktivdestillation eine chemische Umsetzung zu einem Isobutenfolgeprodukt wie MTBE, TBA oder ein Carbonsäureester erfolgt. Dieses Folgeprodukt wird in einer Destillationsstufe aufgereinigt und anschließend in einer zweiten Reaktivdestillation wieder zu Isobuten gespalten. Die Zufuhr des isobutenhaltigen C₄-Kohlenwasserstoffgemischs in die Reaktivdestillationsanlage erfolgt bei diesem Verfahren in die Katalysatorpackung.

Da die bekannten Verfahren hinsichtlich der Raum-Zeit-Ausbeute in der Reaktivdestillationskolonne und/oder der Selektivität und/oder des Isobuten-Restgehalts in der verbleibenden C₄-Fraktion nicht zufriedenstellend sind, bestand die Aufgabe darin, ein Verfahren zu entwickeln, das sich durch eine höhere Raum-Zeit-Ausbeute und nahezu vollständigen Isobuten-Umsatz auszeichnet.

Es wurde nun gefunden, dass die Raum-Zeit-Ausbeute für die Bildung von TBA aus Isobuten und Wasser in Gegenwart eines sauren Katalysators in einer Reaktivdestillationskolonne, und der Isobuten-Umsatz erhöht und der Gehalt an Isobuten im Destillat vermindert werden kann, wenn sowohl das Isobuten-haltige Gemisch als auch Wasser unterhalb der Reaktionszone eingespeist werden.

In EP 0 726 241 ist ein Verfahren zur Spaltung von TBA in Isobuten und Wasser, d. h. die entgegengesetzte Reaktion, mittels Reaktivdestillation offenbart. Auch hier wird das Einsatzgemisch unterhalb der Reaktionszone in die Reaktivdestillationskolonne geleitet. In der reaktiven Zone liegt ein TBA/Isobuten/Wasser-Gemisch vor, das jedoch nicht zu TBA zurückreagiert, sondern fast vollständig zu Isobuten und Wasser umgesetzt wird. Überraschender Weise kann dieses Reaktorkonzept auch zur Bildung von TBA aus Wasser und Isobuten eingesetzt werden.

Gegenstand der vorliegenden Erfindung ist demnach ein Verfahren zur Herstellung von tertiärem Butanol (TBA) durch Umsetzung eines isobutenhaltigen C₄-Kohlenwasserstoffstroms mit Wasser an einem festen sauren Katalysator, wobei in einer ersten Stufe der Isobuten-haltige C₄-Kohlenwasserstoffstrom und Wasser unter Zusatz von TBA in einem oder mehreren Reaktoren zur Reaktion gebracht werden und das erhaltene Gemisch unterhalb der Reaktionszone in eine Reaktivdestillationskolonne eingespeist werden.

In einer Verfahrensvariante wird in die Reaktivdestillationskolonne ein Gemisch aus einem isobutenhaltigen C₄-Kohlenwasserstoffstrom, Wasser und TBA eingespeist. Dieses Gemisch kann durch Reaktion eines isobutenhaltigen C₄-Kohlenwasserstoffstrom mit Wasser und einem festen, sauren Katalysator in einem Vorreaktor erhalten werden.

Bevorzugt wird die Vorreaktion eines isobutenhaltigen C₄-Kohlenwasserstoffstrom mit Wasser an einem sauren festen Katalysator bis zu einem Isobuten-Umsatz von 65 bis 97 % zu tert.-Butanol durchgeführt und das so erhaltene Gemisch in die Reaktivdestillationskolonne eingespeist.

Im erfindungsgemäßen Verfahren ist die Zufuhr des Reaktionsgemisches unterhalb der Reaktionspackung essentiell. Die Zufuhr von Wasser und isobutenhaltigem C₄-Kohlenwasserstoffstrom in die Reaktivdestillationskolonne kann an der gleichen Stelle oder an verschiedenen Stellen erfolgen. Z. B. kann die Zufuhr von Wasser oberhalb des C₄-Kohlenwasserstoffs erfolgen.

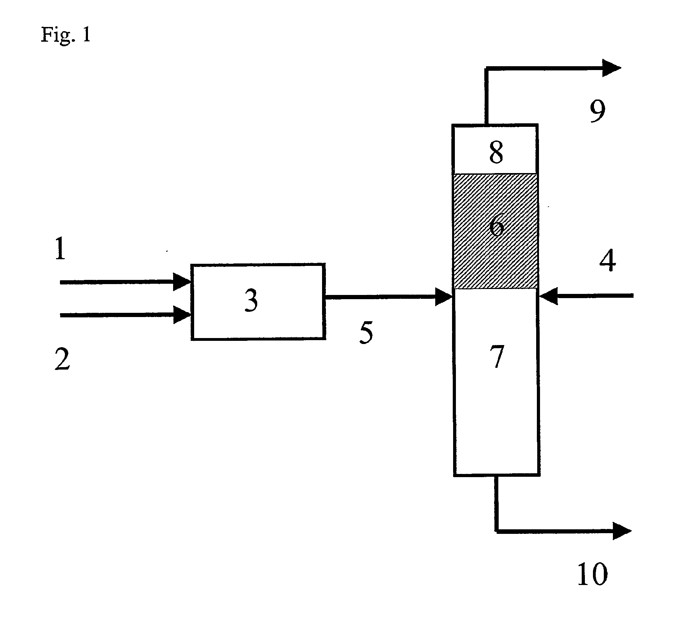

Ein Blockschema einer Anlage, in der TBA nach dem erfindungsgemäßen Verfahren hergestellt werden kann, ist in Figur 1 dargestellt. Das Isobuten-haltige C₄-Kohlenwasserstoffgemisch (1) wird zusammen mit Wasser (2), welches TBA als Lösungsvermittler enthalten kann, in die Reaktoren (3) eingespeist. Die Reaktoren (3) können aus 1 bis 5, vorzugsweise 3 bis 4 Stufen bestehen. Das vorreagierte Gemisch (5) kann mit einem optionalen Wasserstrom (4) in die Reaktivrektifikation eingespeist werden. Die Feedposition für den optionalen Wasserstrom (4) erfolgt im Abtriebsteil der Reaktivrektifikationskolonne. Bevorzugt wird der Wasserstrom (4) auf der gleichen Kolonnenhöhe zugeführt wie das vorreagierte Gemisch (5). In einer besonderen Ausführung des Verfahrens kann dem vorreagiertem Gemisch (5) vor dem Eintritt in die Reaktionskolonne zusätzliches Wasser (4) zugeführt werden, was oberhalb der Sättigungskonzentration auch zu einer Entmischung in eine organische und eine wässrige Phase führen kann.

Optional kann der Vorreaktor entfallen und Strom (1) und Wasser (2 oder 4) werden direkt in die Reaktivdestillationskolonne eingespeist. Die Zulaufpositionen befinden sich unterhalb des reaktiven Bereichs (6). Zwischen Zulauf und Reaktionszone kann sich ein destillativer Bereich befinden. Unterhalb des reaktiven Bereichs befindet sich ein rein destillativer Bereich, welcher zur Abtrennung des TBA und ggf. überschüssigen Wassers dient. Das Sumpfprodukt (10) enthält überwiegend TBA und Wasser, idealerweise mit Wasserkonzentrationen, geringer als im Wasser/TBA-Azeotrop. Optional kann oberhalb des reaktiven Bereichs ein weiterer destillativer Bereich (8) mit 0 bis 10, vorzugsweise 0 bis 6 theoretischen Stufen folgen, um die Isobuten-Konzentration in der Reaktionszone einzustellen. Als Kopfprodukt (9) gewinnt man ein C₄-Gemisch mit geringen Restgehalten an Isobuten und Wasser.

Gewöhnliche Bauteile wie Pumpen, Verdichter, Ventile und Verdampfer sind im Blockschaltbild nicht dargestellt, sind allerdings selbstverständliche Bauteile einer Anlage.

Nach dem erfindungsgemäßen Verfahren kann die Umsetzung von Isobuten mit Wasser zu TBA vorzugsweise in zwei Stufen (siehe Figur 1) durchgeführt werden. Die erste Stufe beinhaltet die Reaktion von Isobuten im C₄-Gemisch mit Wasser, gegebenenfalls unter Zusatz von TBA als Lösungsvermittler, in einem oder mehreren Reaktoren, im Idealfall bis zur Einstellung des thermodynamischen Gleichgewichtes aus TBA, Wasser und Isobuten in einer homogenen Lösung. Die Reaktoren der ersten Stufe können herkömmliche Festbettreaktoren mit den gleichen Katalysatoren sein, die im Folgenden für die zweite Stufe beschrieben werden. Die Reaktoren werden in üblicher Weise bei 30 - 110 °C und 5 - 50 bara betrieben.

Typische Zusammensetzungen der so erhaltenen Reaktionsmischungen sind in den Beispielen beschrieben. In der Regel enthalten diese Gemische unter 20 Massen-%, insbesondere unter 15 Massen-% Isobuten, das in der folgenden zweiten Stufe, in der Reaktivdestillationskolonne, sehr selektiv zu TBA umgesetzt wird.

In der Verfahrensvariante mit Vorreaktor, bei der der isobutenhaltige Stoffstrom durch Umsetzen eines isobutenhaltigen C₄-Kohlenwasserstoffstroms mit Wasser an einem sauren Katalysator hergestellt wird, kann dies auch mit einem begrenzten Umsatz von 65 bis 97 % erfolgen. Bevorzugt sind hier Isobuten-Umsätze von 75 bis 97 %, insbesondere 80 bis 96 %, ganz besonders 82 bis 95 %.

Dies ist z. B. möglich, indem man einen isobutenhaltigen C₄-Kohlenwasserstoffstrom in mehreren Reaktionsstufen an einem sauren Festbettkatalysator umsetzt. Dabei sind die Reaktoren in Reihe und/oder parallel zueinander verschaltet. Die einzelnen Reaktoren werden im geraden Durchgang oder in Schlaufenfahrweise betrieben. Als Reaktionsprodukt wird aus dem Vorreaktor oder Vorreaktorsystem ein Gemisch erhalten, das neben den nicht abreagierten Stoffen aus dem C₄-Kohlenwasserstoffstrom, wie z. B. Isobutan, n-Butan, 1-Buten und 2-Buten, auch Isobuten, tert.-Butanol und Wasser enthält. Die Konzentrationen von Isobuten, tert.-Butanol und Wasser darin liegt nahe beim thermodynamischen Gleichgewicht bei der jeweiligen Temperatur am Reaktorausgang.

Als Einsatzstoffe des erfindungsgemäßen Verfahrens können C₄-Kohlenwasserstoffgemische, die sowohl Isobuten als auch lineare Butene, jedoch keine Acetylenderivate und weniger als 8000 Massen-ppm Butadien enthalten, eingesetzt werden. Technische Gemische, die sowohl Isobuten als auch lineare Butene enthalten können, sind beispielsweise Leichtbenzinfraktionen aus Raffinerien, C₄-Fraktionen aus FCC-Einheiten oder Steamcracker, Gemische aus Fischer-Tropsch-Synthesen, Gemische aus Dehydrierung von Butanen, Gemische aus Skelettisomerisierung linearer Butene, Gemische, entstanden durch Metathese von Olefinen oder anderen technischen Prozessen.

Diese Gemische können nach Entfernung der mehrfach ungesättigten Verbindungen in das erfindungsgemäße Verfahren eingesetzt werden. Beispielsweise kann die Gewinnung eines geeigneten Einsatzstoffsgemisch aus der C₄-Fraktion eines Steamcrackers durch Extraktion des Butadiens oder dessen Selektivhydrierung zu linearen Butenen erfolgen. Dieses Gemisch (Raffinat I bzw. selektivhydriertes Crack-C₄) besteht aus n-Butan, Isobutan, den drei linearen Butenen und Isobuten und ist ein bevorzugtes Edukt für das erfindungsgemäße Verfahren.

Wird der isobutenhaltige Stoffstrom mit Wasser in die Reaktivdestillationskolonne unterhalb der reaktiven Packung eingespeist, so steigen die leichtsiedenden C₄-Kohlenwasserstoffe dampfförmig in die Reaktionszone auf und aufgrund des Minimum-Azeotrop von Wasser und C₄-Kohlenwasserstoffen wird mit dem Edukt auch ein Teil des Wassers dampfförmig in die Reaktionszone transportiert. Das gegebenenfalls im Einsatzstoffgemisch enthaltene TBA und Teile des Wassers verbleiben im Sumpf und werden standgeregelt abgeführt.

Die im Azeotrop enthaltenen C₄-Kohlenwasserstoffe bestehen bei Einsatz eines C₄-Kohlenwasserstoffgemischs, wie z. B. Raffinat I, aus Isobuten, Isobutan, n-Butan, 1-Buten und 2-Butene. Isobuten und Wasser werden in der Reaktivzone der Kolonne zum TBA umgesetzt, das als Schwersieder in den Sumpf der Kolonne fließt. Die übrigen Bestandteile des Azeotrops reagieren unter den Reaktionsbedingungen nicht mit Wasser und werden am Kopf der Kolonne als wasserhaltiges Azeotrop abgetrennt.

Im Idealfall enthält das am Kopf der Reaktivdestillationskolonne abgetrennte Gemisch kein oder nahezu kein Isobuten mehr.

In einer weiteren Verfahrensvariante wird das am Kopf der Reaktivdestillationskolonne erhaltene Gemisch in eine wässrige und eine organische Phase getrennt und die wässrige Phase in die Reaktivdestillationskolonne zurückgeführt.

Diese Verfahrensvariante hat den Vorteil, dass das Reaktionswasser weitgehend im Kreis geführt wird und das Kopfprodukt der Kolonne nur noch geringe Mengen an Wasser enthält.

Optional kann ein Teil der organischen Destillatphase oder ein Teil des gesamten Destillats in die Reaktivdestillationskolonne zurückgeführt werden.

Die Rückführung der wässrigen Destillatphase kann oberhalb und/oder unterhalb, die der organischen Destillatphase oberhalb der Reaktionszone erfolgen.

Die Reaktivdestillationskolonne enthält in der Verstärkersäule den Katalysator, unter- und oberhalb der Katalysatorpackung befinden sich Trennböden oder Destillationspackungen. Der Katalysator ist entweder in einer Packung integriert, beispielsweise KataMax^{®} (EP 0 428 265), KataPak^{®} (EP 0 396 650) oder MultiPak^{®}(Gebrauchsmuster Nr. 298 7 007.3), oder auf Formkörper aufpolymerisiert (US 5 244 929). Bevorzugt werden katalytische Packungen mit einem hohen Katalysatorgehalt eingesetzt, wie z. B. Katapak-SP 12 oder besonders bevorzugt Katapak-SP 11.

Unter dem Oberbegriff der Reaktivdestillation fallen alle verfahrenstechnischen Maßnahmen, bei denen Destillation und Reaktion gleichzeitig durchgeführt werden. In den beschriebenen Reaktoren ist dies durch eine besondere Ausführung der Packungen in einer Kolonne erreicht. Es ist im erfindungsgemäßen Verfahren auch möglich, diese Bereiche räumlich zu trennen, ohne auf die Vorteile einer Reaktivdestillation zu verzichten.

In einer Verfahrensvariante wird die Reaktivdestillationskolonne als Destillationskolonne mit einem oder mehreren außenliegenden Reaktoren, die den Katalysator enthalten und im Nebenstrom betrieben werden, ausgeführt.

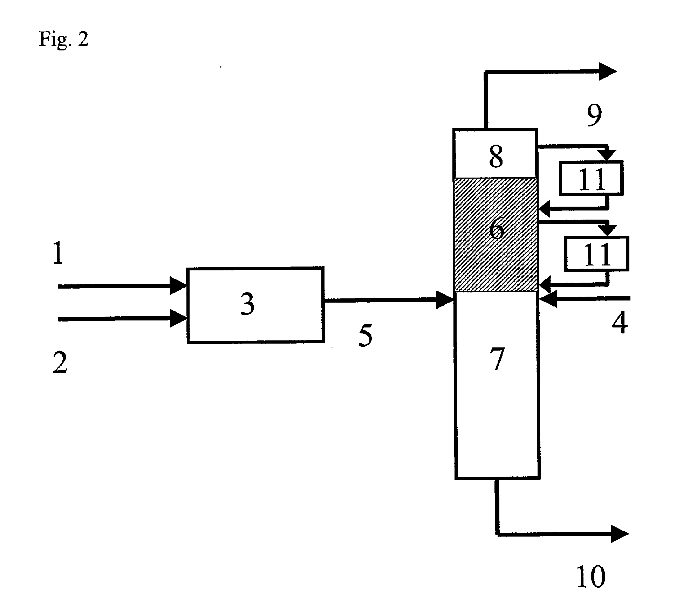

Der Katalysator kann, wie in Figur 2 gezeigt, ganz oder teilweise in außenliegenden Reaktoren (11) installiert sein. Auf diese Weise lässt sich eine größere Katalysatormenge einsetzen und einen Katalysatorwechsel deutlich vereinfachen.

In einer weiteren Variante ist die Reaktivdestillationskolonne als Destillationskolonne mit einem oder mehreren im Destillationsteil integrierten Reaktoren, die den Katalysator enthalten, ausgeführt.

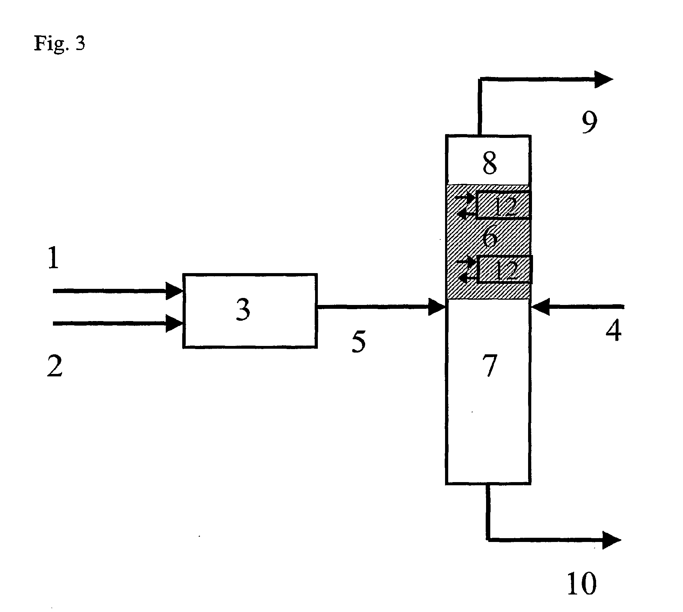

Hier kann der Katalysator, wie in Figur 3 gezeigt, ganz oder teilweise in integrierten Reaktoren (12) installiert sein. Als integrierten Reaktor oder integrierte Reaktionsstufe ist beispielsweise eine Katalysatorschüttung zu verstehen, welche nur von flüssiger Phase durchströmt wird und somit intensiven Edukt-Katalysator-Kontakt ermöglicht. Konstruktiv lässt sich dies beispielsweise wie beim Catacol-Prozess der IFP US 5,776320 oder auch als Katalysatorschüttung in Ablaufschächten eines Destillationsbodens realisieren. Auf diese Weise lässt sich ebenfalls eine größere Katalysatormenge einsetzen.

Als eigentlicher Katalysator wird in beiden Stufen des Verfahrens ein fester Stoff, der weder im Einsatzstoffgemisch noch im Produktgemisch löslich ist, mit sauren Zentren an seiner Oberfläche eingesetzt. Der Katalysator darf unter Reaktionsbedingungen keine sauren Stoffe an das Produktgemisch abgeben, weil dies zu Ausbeuteverlusten führen würde.

Für die Aktivität der Katalysatoren gilt, dass sie unter Reaktionsbedingungen die Addition von Wasser an Isobuten bewirken, jedoch kaum die Addition an lineare Butene. Weiterhin dürfen sie die Oligomerisierung von Olefinen kaum katalysieren.

Eine im Verfahren der Erfindung einsetzbare Gruppe von sauren Katalysatoren sind feste Ionenaustauscherharze mit Sulfonsäuregruppen. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden. Stark saure Harze des Styrol-Divinyl-Typs werden u. a. unter folgenden Handelsnamen verkauft: Duolite C20, Duolite C26, Amberlyst 15, Amberlyst 35, Amberlite IR-120, Amberlite 200, Dowex 50, Lewatit SPC 118, Lewatit SPC 108, Lewatit K2621, Lewatit K2629, Lewatit K2431.

Die Eigenschaften dieser Harze, insbesondere spezifische Oberfläche, Porosität, Stabilität, Quellung bzw. Schrumpfung und Austauschkapazität, können durch den Herstellprozess variiert werden.

Im erfindungsgemäßen Verfahren können die Ionenaustauscherharze in ihrer H-Form eingesetzt werden. Es werden vorzugsweise makroporöse Harze eingesetzt, wie beispielsweise Lewatit SCP 118, Lewatit SCP 108, Amberlyst 15 oder Amberlyst 35, Lewatit K2621, Lewatit K2629, Lewatit K2431. Das Porenvolumen beträgt 0,3 bis 0,9 ml/g, insbesondere 0,5 bis 0,9 ml/g. Die Korngröße des Harzes liegt zwischen 0,3 mm und 1,5 mm, insbesondere zwischen 0,5 mm und 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Säurekapazität des Ionenaustauscher beträgt, bezogen auf Lieferform, 0,7 - 2,0 eq/l, insbesondere 1,1-2,0 eq/l.

Die destillative Trennung in der Reaktivrektifikation wird mit Einbauten, die aus Böden, rotierenden Einbauten, ungeordneten und/oder geordneten Packungen bestehen, durchgeführt.

Bei den Kolonnenböden kommen folgende Typen zum Einsatz:
- Böden mit Bohrungen oder Schlitzen in der Bodenplatte.
- Böden mit Hälsen oder Kaminen, die von Glocken, Kappen oder Hauben überdeckt sind.
- Böden mit Bohrungen in der Bodenplatte, die von beweglichen Ventilen überdeckt sind.
- Böden mit Sonderkonstruktionen.

In Kolonnen mit rotierenden Einbauten wird der Rücklauf entweder durch rotierende Trichter versprüht oder mit Hilfe eines Rotors als Film auf einer beheizten Rohrwand ausgebreitet.

In dem erfindungsgemäßen Verfahren verwendete Kolonnen können regellose Schüttungen mit verschiedenen Füllkörpern enthalten. Sie können aus fast allen Werkstoffen - Stahl, Edelstahl, Kupfer, Kohlenstoff, Steingut, Porzellan, Glas, Kunststoffen usw. - und in verschiedenen Formen - Kugeln, Ringen mit glatten oder profilierten Oberflächen, Ringen mit Innenstegen oder Wanddurchbrüchen, Drahtnetzringen, Sattelkörper und Spiralen - bestehen.

Packungen mit regelmäßiger Geometrie können z.B. aus Blechen oder Geweben bestehen. Beispiele solcher Packungen sind Sulzer Gewebepackungen BX aus Metall oder Kunststoff, Sulzer Lamellenpacktingen Mellapak aus Metallblech, Hochleistungspackungen wie MellapakPlus, Strukturpackungen von Sulzer (Optiflow), Montz (BSH) und Kühni (Rombopak).

Die Reaktivrektifikationskolonne, in der Isobuten umgesetzt und in der ein TBA-reicher Strom als Sumpfprodukt abgezogen wird, hat eine Trennstufenzahl von 2 bis 60 insbesondere von 3 bis 50. Dabei entfallen 5 bis 58 Trennstufen auf den Abtriebsteil, 2 bis 55 Trennstufen auf die Reaktionszone und 0 bis 20 Trennstufen auf den Verstärkerteil über der Reaktionszone. Die Zulaufposition befindet sich unterhalb des reaktiven Bereichs. Wasser und vorreagiertes Gemisch werden bevorzugt an gleicher Position eingeleitet.

Der Betriebsdruck der Reaktivdestillationskolonne, gemessen am Kolonnenkopf, liegt zwischen 3 und 30 bara, insbesondere zwischen 4 und 12 bara. Das Rücklaufverhältnis liegt im Bereich von 0,5 bis 40, insbesondere im Bereich von 0,9 bis 20.

Das Einsatzkohlenwasserstoffgemisch kann zusammen mit Wasser und optional TBA als Lösungsvermittler in die Vorreaktoren eingespeist werden. Als Katalysatoren werden die gleichen wie in der Reaktivdestillationskolonne verwendet. Bevorzugt wird die Vorreaktion in 2, 3, 4 oder 5 Stufen durchgeführt. Dabei entsteht ein Gemisch aus TBA, Wasser, Isobuten und anderen Kohlenwasserstoffen.

Das erfindungsgemäße Verfahren kann mit einem homogenen, d. h. mit einem mit Wasser gesättigten Gemisch, oder mit einem heterogenen Gemisch durchgeführt werden.

Wird das Verfahren zweistufig (Vorreaktion und Umsetzung in einer Reaktivdestillationskolonne) ausgeführt, so kann im Zulauf der Reaktivdestillationskolonne mehr Wasser enthalten sein, als für die vollständige Umsetzung des noch vorhandenen Isobutens gebraucht wird. Ist im Reaktionsgemisch der ersten Stufe zu wenig Wasser enthalten, sollte zusätzliches Wasser in die Reaktivdestillationskolonne geführt werden.

Im erfindungsgemäßen Verfahren fällt ein Destillat an, das bevorzugt weniger als 700, insbesondere weniger als 450 Massen-ppm Isobuten, ganz besonders weniger als 400 Massen-ppm enthält. Aus dem so erhaltenen Raffinat II können Spuren von Butadien durch Selektivhydrierung entfernt werden. Dieses Gemisch kann destillativ in Isobutan, 1-Buten und ein Gemisch aus 2-Butenen und n-Butan oder in Isobutan und ein Gemisch aus linearen Butenen und n-Butan getrennt werden.

Bei der Aufarbeitung eines Destillats mit weniger als 450 Massen-ppm Isobuten kann ein 1-Buten mit weniger als 1000 Massen-ppm Isobuten gewonnen werden, das ein gefragtes Zwischenprodukt ist. Es wird beispielsweise als Comonomer bei der Herstellung von Polyethylen (LLDPE oder HDPE) sowie von Ethylen-Propylen-Mischpolymeren eingesetzt. Es findet weiterhin Einsatz als Alkylierungsmittel und ist Ausgangsstoff für die Herstellung von Butan-2-ol, Butenoxid, Valeraldehyd.

Eine weitere Verwendung des erfindungsgemäß hergestellten nahezu isobutenfreien, Raffinats II ist die Herstellung von n-Buten-Oligomeren, insbesondere nach dem Octol-Prozess.

Die nach Abtrennung bzw. Umsetzung der linearen Butene aus dem Raffinat II zurückbleibenden Kohlenwasserstoffe können gegebenenfalls nach Hydrierung (CSP) zu Isobutan und n-Butan aufgearbeitet werden.

Das Sumpfprodukt, das TBA, Wasser und Hochsieder, wie C₈-Olefine, enthält, kann direkt verwendet werden oder aufgearbeitet werden. Nach bekannten Verfahren kann daraus TBA und TBA/Wasser-Azeotrop oder nur TBA hergestellt werden. Wasserarme TBA-Qualitäten können als Kraftstoffkomponente verwendet werden.

Der Vorteil des erfindungsgemäßen Verfahrens besteht darin, dass im Vergleich zu herkömmlichen Verfahren ein hoher Umsatz an Isobuten erreicht werden kann, ohne dass große Reaktorvolumina erforderlich sind. Dies wird nur durch die kombinierte Stoffabtrennung und Reaktion in der Reaktivrektifikation möglich.

Die folgenden Beispiele sollen die Erfindung erläutern, ohne ihre Anwendungsbreite einzuschränken, die sich aus der Beschreibung und den Patentansprüchen ergibt.

### Beispiele:

Der für die Versuche eingesetzte Raffinatstrom hatte die Zusammensetzung:

| | |
|---|---|
| n-Butan: | 8,2 % |
| Iso-Butan: | 2,3 % |
| 1-Buten: | 29,8 % |
| 2-Buten (cis + trans): | 14,2 % |
| Isobuten: | 45,5 % |

Der Isobuten-Gehalt im Raffinat liegt typischerweise im Bereich von 35 bis 55 %.

### 1. Beispiel (gemäß der Erfindung)

Die Herstellung von tert.-Butanol erfolgte in einer nach Figur 1 realisierten Anlage, wobei zur Vereinfachung zunächst nur die Reaktivdestillation untersucht wurde.
Der Kolonnendurchmesser der Reaktivrektifikationskolonne betrug dabei 80 mm. Im unteren Teil der Kolonne waren 10 Laborglockenböden als destillativer Abtriebsteil (7) realisiert, darüber befand sich der reaktive Teil der Kolonne (6), der über eine Höhe von 3000 mm mit Katapak-SP 12-Elementen der Firma Sulzer bestückt war. Der Zulauf erfolgte unterhalb des Reaktionsteils der Kolonne und bestand aus vorreagiertem Gemisch (5) sowie einem zusätzlichen Wasserstrom (4). Die Stromnummern in der folgenden Tabelle korrespondierten mit der Darstellung in Figur 1. Prozentangaben sind als Massen-% zu lesen. Als Katalysator wurde Amberlyst 35 eingesetzt. Der Katalysatoranteil in der reaktiven Zone betrug 25 Vol.-%.

| Stromnummer | Strombezeichnung | Massenfluss [kg/h] | Zusammensetzung |
|---|---|---|---|
| 5 | Vorreaktorausgang | 5,5 | 5,9 % Isobuten |
| | | | 49,1 % TBA |
| | | | 5,5 % Wasser |
| | | | 39,5 % Rest-C₄ |
| 4 | Frisch-Wasser | 0,05 | |
| 9 | Destillat | 2,2 | 403 Massen-ppm Isobuten |
| | | | 1,0 % Wasser |
| | | | 99,0 % Rest-C₄ |
| 10 | Sumpfprodukt | 3,35 | 6,8 % Wasser |
| | | | 93,0 % TBA |
| | | | 0,1 % C₈ (Hochsieder) |

Der Druck der Reaktivrektifikationskolonne betrug 7 bar. Das Rücklaufverhältnis lag bei 15.

### 2. Beispiel (gemäß der Erfindung)

Die Herstellung von tert.-Butanol erfolgte in einer vollständig nach Figur 1 realisierten Anlage.
Als Vorreaktoren wurden 3 Laborreaktoren mit Amberlyst 35 verwendet. Das Reaktionswasser wurde stufenweise zugegeben. Der erste Reaktor hatte ein Katalysatorvolumen von 1 1 und wurde mit einem äußeren Kreislauf von 3,0 kg/h bei einer Eingangstemperatur von 60 °C betrieben. Der Raff. I-Frisch-Zulauf (1) betrug 1 kg/h, die Prozesswassermenge betrug 0,08 kg/h (2). Der zweite Reaktor hatte ebenfalls ein Katalysatorvolumen von 1 1 und wurde im einfachen Durchgang bei einer Eingangstemperatur von 55 °C betrieben. Die Prozesswassermenge für den zweiten Reaktor betrug 0,05 kg/h (2a). Der dritte Reaktor hatte ein Katalysatorvolumen von 1 1 und wurde im einfachen Durchgang bei einer Eingangstemperatur von 55 °C betrieben. Die Prozesswassermenge für den dritten Reaktor betrug 0,043 kg/h (2b). Der Druck der Anlage wurde am Eingang zum ersten Reaktor auf 11,6 bar eingestellt. Um genügend Produkt für die nachfolgende Destillation zu gewinnen, wurde das Produkt der Reaktoren zunächst gesammelt und dann als Zulaufstrom zur Kolonne eingesetzt.

Der Kolonnendurchmesser der Reaktivrektifikationskolonne betrug dabei 80 mm. Im unteren Teil der Kolonne waren 15 Laborglockenböden als destillativen Abtriebsteil (7) realisiert, darüber saß der reaktive Teil der Kolonne (6), der über eine Höhe von 3000 mm mit Katapak-SP 12-Elementen der Firma Sulzer bestückt war. Der Zulauf zur Kolonne erfolgte unterhalb des Reaktionsteils der Kolonne und bestand aus vorreagiertem Gemisch (5) ohne einen zusätzlichen Wasserstrom (4). Die Stromnummern in der folgenden Tabelle korrespondieren mit der Darstellung in Figur 1. Prozentangaben sind als Massen-% zu lesen. Als Katalysator wurde Amberlyst 35 eingesetzt. Der Katalysatoranteil in der reaktiven Zone betrug 25 Vol-%.

| Stromnummer | Strombezeichnung | Massenfluss [kg/h] | Zusammensetzung |
|---|---|---|---|
| 1 | Raffinat-I-Frisch-Zulauf | 1,0 | 45,5 % Isobuten |
| | | | 54,5 % Rest-C₄ |
| 2 | Prozesswasser | 0,34 | 49,3 % TBA |
| | | | 50,7 % Wasser |
| 4 | Zusatzwasser für Kolonne | 0 | |
| | | | |
| 5 | Zulauf zur Kolonne | 5,0 | 5,6 % Isobuten |
| | | gesammeltes Produkt aus dem dritten Reaktor | 48,7 % TBA |
| | | | 3,8 % Wasser |
| | | | 40,5 % Rest-C₄ |
| | | | 1,4 % Sonstige |
| 9 | Destillat | 2,04 | 343 Massen-ppm Isobuten |
| | | | 1,0 % Wasser |
| | | | 98,9 % Rest-C₄ |
| 10 | Sumpfprodukt | 2,96 | 2,8 % Wasser |
| | | | 95,2 % TBA |
| | | | 2,0 % Sonstige (Hochsieder) |

Der Druck der Reaktivrektifikationskolonne betrug 7 bar. Das Rücklaufverhältnis lag bei 15.

### 3. Beispiel (Vergleich)

Die Herstellung von tert.-Butanol erfolgte in einer nach Figur 1 realisierten Anlage, mit den Bedingungen aus Beispiel 1, wobei nur die Zufuhr des Wassers geändert und oberhalb des Reaktionsteils erfolgte. Der erreichte Umsatz war schlechter, wie in der folgenden Tabelle anhand der Isobuten-Konzentrationen im Destillat (9) zu erkennen ist.

| Stromnummer | Strombezeichnung | Massenfluss [kg/h] | Zusammensetzung |
|---|---|---|---|
| 5 | Vorreaktorausgang | 5,1 | 5,9 % Isobuten |
| | | | 49,1 % TBA |
| | | | 5,5 % Wasser |
| | | | 39,5 % Rest-C₄ |
| 4 | Frisch-Wasser | 0,5 | |
| 9 | Destillat | 2,05 | 0,9 % Isobuten (9000 ppm) |
| | | | 1,1 % Wasser |
| | | | 98,0 % Rest-C4 |
| 10 | Sumpfprodukt | 3,1 | 6,9 % Wasser |
| | | | 93,0 % TBA |
| | | | 0,1 % Sonstige (Hochsieder) |

Die Beispiele belegen, dass im erfindungsgemäßen Verfahren, bei dem sowohl der Isobutenhaltige Strom als auch gegebenenfalls Wasser unterhalb der reaktiven Zone eingespeist wird, ein höherer Umsatz an Isobuten und somit ein Destillat mit geringerem Isobuten-Gehalt erhalten wird, als bei einem herkömmlichen Verfahren.

## Patentansprüche

1. Verfahren zur Herstellung von tertiärem Butanol (TBA) durch Umsetzung eines isobutenhaltigen C₄-Kohlenwasserstoffstroms mit Wasser an einem festen sauren Katalysator,
**dadurch gekennzeichnet,**
**dass** in einer ersten Stufe der Isobuten-haltige C₄-Kohlenwasserstoffstrom und Wasser unter Zusatz von TBA in einem oder mehreren Reaktor/-en zur Reaktion gebracht werden und das erhaltene Gemisch unterhalb der Reaktionszone in eine Reaktivdestillationskolonne eingespeist wird.

2. Verfahren nach Anspruch 1,
**dadurch gekennzeichnet,**
**dass** ein isobutenhaltiger C₄-Kohlenwasserstoffstrom, Wasser und tert.-Butanol in die Reaktivdestillationskolonne eingespeist werden.

3. Verfahren nach Anspruch 1 oder 2,
**dadurch gekennzeichnet,**
**dass** zunächst ein isobutenhaltiger C₄-Kohlenwasserstoffstrom mit Wasser an einem sauren festen Katalysator bis zu einem Isobuten-Umsatz von 65 bis 97 % zu tert.-Butanol umgesetzt und das so erhaltene Gemisch in die Reaktivdestillationskolonne eingespeist wird.

4. Verfahren nach einem der Ansprüche 1 bis 3,
**dadurch gekennzeichnet,**
**dass** das am Kopf der Reaktivdestillationskolorine erhaltene Gemisch in eine wässrige und eine organische Phase getrennt und die wässrige Phase in die Reaktivdestillationskolonne zurückgeführt wird.

5. Verfahren nach einem der Ansprüche 1 bis 4,
**dadurch gekennzeichnet,**
**dass** die Reaktivdestillationskolonne als Destillationskolonne mit einem oder mehreren außenliegenden Reaktoren, die den Katalysator enthalten und im Nebenstrom betrieben werden, ausgeführt ist.

6. Verfahren nach einem der Ansprüche 1 bis 4,
**dadurch gekennzeichnet,**
**dass** die Reaktivdestillationskolonne als Destillationskolonne mit einem oder mehreren im Destillationsteil integrierten Reaktoren, die den Katalysator enthalten, ausgeführt ist.

7. Verfahren nach einem der Ansprüche 1 bis 6,
**dadurch gekennzeichnet,**
**dass** in die Reaktivdestillationskolonne ein homogenes, mit Wasser gesättigtes Gemisch eingespeist wird.

8. Verfahren nach einem der Ansprüche 1 bis 6,
**dadurch gekennzeichnet,**
**dass** in die Reaktivdestillationskolonne ein heterogenes Gemisch eingespeist wird.

9. Verfahren nach einem der Ansprüche 1 bis 8,
**dadurch gekennzeichnet,**
**dass** die Einspeisung des isobutenhaltigen C₄-Kohlenwasserstoffstroms und Wasser an verschiedenen Stellen der Reaktivdestillationskolonne erfolgt.

## Claims

1. Process for preparing tertiary butanol (TBA) by reacting an isobutene-containing C₄-hydrocarbon stream with water over a solid acidic catalyst,
**characterized in that**
the isobutene-containing C₄-hydrocarbon stream and water are reacted in a first stage in one or more reactor(s) with the addition of TBA and the mixture obtained is fed into a reactive distillation column below the reaction zone.

2. Process according to Claim 1,
**characterized in that**
an isobutene-containing C₄-hydrocarbon stream, water and tert-butanol are fed into the reactive distillation column.

3. Process according to Claim 1 or 2,
**characterized in that**
an isobutene-containing C₄-hydrocarbon stream is firstly reacted with water over an acidic solid catalyst to a conversion of isobutene into tert-butanol of from 65 to 97% and the mixture obtained in this way is fed into the reactive distillation column.

4. Process according to any of Claims 1 to 3,
**characterized in that**
the mixture obtained at the top of the reactive distillation column is separated into an aqueous phase and an organic phase and the aqueous phase is returned to the reactive distillation column.

5. Process according to any of Claims 1 to 4,
**characterized in that**
the reactive distillation column is configured as a distillation column provided with one or more external reactors in which the catalyst is present and through which a secondary stream passes.

6. Process according to any of Claims 1 to 4,
**characterized in that**
the reactive distillation column is configured as a distillation column provided with one or more catalyst-containing reactors integrated into the distillation section.

7. Process according to any of Claims 1 to 6,
**characterized in that**
a homogeneous mixture saturated with water is fed into the reactive distillation column.

8. Process according to any of Claims 1 to 6,
**characterized in that**
a heterogeneous mixture is fed into the reactive distillation column.

9. Process according to any of Claims 1 to 8,
**characterized in that**
the isobutene-containing C₄-hydrocarbon stream and water are fed in at different points on the reactive distillation column.

## Revendications

1. Procédé de préparation de butanol tertiaire (TBA) par réaction d'un courant d'hydrocarbures en C₄, contenant de l'isobutène, avec de l'eau, sur un catalyseur acide solide, **caractérisé en ce que**, dans une première étape, on fait réagir le courant d'hydrocarbures en C₄ contenant de l'isobutène et de l'eau, avec addition de TBA, dans un ou plusieurs réacteur(s), et que le mélange obtenu est introduit dans une colonne de distillation réactive au-dessous de la zone de réaction.

2. Procédé suivant la revendication 1, **caractérisé en ce que** l'on introduit dans la colonne de distillation réactive un courant d'hydrocarbures en C₄ contenant de l'isobutène, de l'eau et du tert.-butanol.

3. Procédé suivant la revendication 1 ou 2, **caractérisé en ce que** l'on fait d'abord réagir un courant d'hydrocarbures en C₄ contenant de l'isobutène avec de l'eau, sur un catalyseur acide solide, jusqu'à un taux de conversion de l'isobutène de 65 à 97%, pour former du tert.-butanol, et que le mélange ainsi obtenu est introduit dans une colonne de distillation réactive.

4. Procédé suivant l'une quelconque des revendications 1 à 3, **caractérisé en ce que** le mélange obtenu en tête de la colonne de distillation réactive est séparé en une phase aqueuse et une phase organique, et que la phase aqueuse est renvoyée dans la colonne de distillation réactive.

5. Procédé suivant l'une quelconque des revendications 1 à 4, **caractérisé en ce que** la colonne de distillation réactive est configurée en colonne de distillation avec un ou plusieurs réacteurs extérieurs, qui contiennent le catalyseur et sont conduits en courant secondaire.

6. Procédé suivant l'une quelconque des revendications 1 à 4, **caractérisé en ce que** la colonne de distillation réactive est configurée en colonne de distillation avec un ou plusieurs réacteurs intégrés à la partie distillation, et qui contiennent le catalyseur.

7. Procédé suivant l'une quelconque des revendications 1 à 6, **caractérisé en ce que** l'on introduit dans la colonne de distillation réactive un mélange homogène saturé d'eau.

8. Procédé suivant l'une quelconque des revendications 1 à 6, **caractérisé en ce que** l'on introduit dans la colonne de distillation réactive un mélange hétérogène.

9. Procédé suivant l'une quelconque des revendications 1 à 8, **caractérisé en ce que** l'introduction du courant d'hydrocarbures en C₄ contenant de l'isobutène et de l'eau se fait en différents endroits de la colonne de distillation réactive.
